# EUROPEAN PATENT APPLICATION

(11) **EP 4 043 586 A1**
(43) Date of publication of application: **17.08.2022**
(21) Application number: 20874860.8
(22) Date of filing: 29.09.2020
(51) Int. Cl.: C12Q 1/6881, C12Q 1/6886

(54) **METHOD FOR DETERMINING WHETHER BIOLOGICAL SAMPLE HASORIGINATED FROM LIVER CANCER TISSUE**

(30) Priority: 08.10.2019 KR 20190124552
(71) Applicant: Lepidyne Co., Ltd., Seongdong-gu Seoul 04779 (KR)
(72) Inventor: KIM, Young Joon, Seoul 06024 (KR); KIM, Da Won, Seoul 06366 (KR); HA, Jeong Sil, Seoul 06024 (KR); KIM, Ji Won, Seongnam-si Gyeonggi-do 13332 (KR)
(74) Representative: Grünecker Patent- und Rechtsanwälte PartG mbB
(86) International application number: PCT/KR2020/013288
(87) International publication number: WO 2021/071163

(57) **Abstract**

The present invention relates to a method for determining whether a biological sample of unknown origin is derived from liver cancer tissue and a composition comprising a liver cancer tissue-specific DNA methylation marker for performing the same, and the liver cancer tissue-specific DNA methylation marker has a low methylation level in normal liver tissue and other tissue, and has a high methylation level only in liver cancer tissue, and thus, it can determine whether a biological sample is derived from liver cancer tissue with excellent accuracy.

## Description

### [TECHNICAL FIELD]

The present invention relates to a method for determining whether a biological sample of unknown origin is derived from liver cancer tissue, and a composition comprising a DNA methylation marker specific to liver cancer tissue for performing the same.

### [BACKGROUND ART]

Cells in the human body have the same genetic information, but the functions and shapes of each cell are very diverse. This is because specific genes are expressed in each cell, and accordingly, the cell differentiation process is different, resulting in a difference in cell phenotype. Several factors, such as DNA methylation, histone modification, tissue-specific transcription factors are involved in expression of these specific genes. In particular, DNA methylation, specifically, methylation of the CpG site, is an essential element for cell-specific gene expression and is known to have unique DNA methylation characteristics for each cell or tissue. Therefore, the origin of a cell or tissue can be easily identified by using DNA methylation characteristics.

Identification of the origin of a cell or tissue can enable early diagnosis of diseases, and increase the accuracy of diagnosis. For example, circulating cell free DNA (cfDNA) is present in blood circulating in a living body, and among them, there may be circulating tumor DNA (ctDNA) flowed out from tumor cells. Even if the presence of ctDNA is detected by a liquid biopsy, in order to accurately diagnose cancer, it is necessary to additionally confirm the tissue from which the ctDNA originated, and in this process, diagnosis of cancer is delayed. However, early diagnosis of cancer is possible if the tissue from which it originated can be identified while detecting the corresponding ctDNA, and a method for identifying the origin of a biological sample is increasingly needed for early diagnosis of other diseases as well as cancer.

The present inventors have studied a method for identifying the origin of a tissue and cell of unknown origin based on DNA methylation data, and as a result, have confirmed a marker with a high methylation level specifically for liver cancer tissue, thereby completing the present invention.

### [DISCLOSURE]

### [TECHNICAL PROBLEM]

An object of the present invention is to provide a method for determining whether a biological sample of unknown origin is derived from liver cancer tissue, a method for confirming liver cancer tissue-derived DNA in a biological sample, and a composition for performing the methods.

### [TECHNICAL SOLUTION]

In order to achieve the above object, one aspect of the present invention provides a method for determining whether a biological sample originates from liver cancer tissue comprising the following steps:
(a) separating DNA from a biological sample isolated from a subject; and
(b) measuring a methylation level of CpG site of the sequence represented by SEQ ID NO: 1 in the separated DNA.

In one specific embodiment of the present invention, the step (b) may further measure a methylation level of CpG site of the sequence represented by SEQ ID NO: 2 in the separated DNA. For example, the method for confirming whether a biological sample originates from liver cancer tissue of the present invention may measure the methylation level of CpG site of the sequence represented by SEQ ID NO: 1 alone, or SEQ ID NO: 1 and 2 in the step (b).

In one specific embodiment of the present invention, the subject may be a human, and the biological sample comprises tissue, tissue fragments, cells, cell fragments, blood, plasma, body fluids, feces and urine isolated from the subject, and the like, but not limited thereto. The tissue, tissue fragments, cells and cell fragments, and the like may be separated from the blood, plasma, body fluids, urine and the like collected from the subject. In addition, the DNA may be DNA isolated from tissue, cells, and the like, and may be cell free DNA (cfDNA) floating in the blood, plasma, body fluids and the like or circulating tumor DNA (ctDNA) flowed out of tumor cells.

The term used in the present specification, "methylation" means attachment of a methyl group (-CH₃) to a base constituting DNA, and preferably, means methylation occurring at a cytosine of specific CpG site in specific DNA.

The term used in the present specification, "methylation level" refers to quantitative evaluation of methylation status of CpG site present in specific DNA sequence, and the methylation status refers to the presence or absence of 5-methyl-cytosine at one or more CpG sites in the DNA sequence.

The term used in the present specification, "CpG site" refers to a sequence in which cytosine (C) and guanine (G) are linked by a phosphate group, and it may be present in a DNA sequence comprising a promoter region, a protein coding region (open reading frame, ORF) and a terminator region, and the like. The methylation of CpG site is known to involve in maintaining genome stability and regulating gene expression, and the like.

The present inventors have tried to discover a liver cancer tissue-specific methylation marker, and as a result, have discovered cg13204512 (SEQ ID NO: 1) and cg00108164 (SEQ ID NO: 2) markers which has high methylation level in liver cancer tissue sample and has low methylation level in other tissues including normal liver tissue and blood. The target methylation sites of the two markers are CpG sites positioned in the 61 st nucleotide in the sequence represented by SEQ ID NO: 1 and SEQ ID NO: 2, respectively. However, as methylation may occur even in other CpG sites in addition to the target methylation sites, in the present invention, the methylation level of the total CpG sites present in the sequences represented by SEQ ID NO: 1 and 2 comprising the target CpG sites can be measured.

The term used in the present specification, "liver cancer tissue-specific methylation marker" means a methylation marker showing a specific methylation level only in DNA of liver cancer tissue compared to a methylation level of DNA isolated from normal liver tissue and other tissue. In the present invention, the "liver cancer tissue-specific methylation marker" means a marker with a high methylation level only in DNA of liver cancer tissue compared to a methylation level of DNA isolated from normal liver tissue and other tissue.

In one specific embodiment of the present invention, the (b) may be performed by a method selected from the group consisting of PCR, methylation specific PCR, real time methylation specific PCR, MethyLight PCR, MehtyLight digital PCR, EpiTYPER, PCR using methylated DNA specific binding protein, quantitative PCR, DNA chip, molecular beacon, MS-HRM (Methylation-sensitive high resolution melting), asymmetric PCR, asymmetric PCR MS-HRMA (asymmetric PCR Methylation-sensitive high resolution melting analysis), Recombinase Polymerase Amplification, LAMP (Loop-Mediated Isothermal Amplification), Eclipse probe, next generation sequencing panel (NGS panel), pyrosequencing and bisulfide sequencing.

For example, the methylation level may be identified by microarray, and the microarray may use a probe fixed on a solid surface. The probe may comprise a sequence complementary to 10 to 100 continuous nucleotide sequences comprising the CpG site.

In one specific embodiment of the present invention, the method may further comprise (c) comparing the methylation level to a methylation level of a control group, after the (b).

In the present invention, the control group means DNA isolated from a biological sample already known which tissue it originated from, and all tissues in a living body including normal liver tissue and liver cancer tissue may be used. For example, in case of using liver cancer tissue as a control group, when the methylation level of DNA isolated from a biological sample is similar to the methylation level of DNA isolated from the control group, it can be determined that the biological sample is derived from liver cancer tissue, and in case of using normal liver tissue or other tissue as a control group, when the methylation level of a biological sample is higher than the methylation level of the control group, it can be determined that the biological sample is derived from liver cancer tissue.

Another aspect of the present invention provides a composition for determining whether a biological sample originates from liver cancer tissue comprising an agent capable of measuring a methylation level of the sequence represented by SEQ ID NO: 1.

In one specific embodiment of the present invention, the CpG site may be 1 to a plurality comprising CpG site positioned at the 61st nucleotide in the sequences represented by SEQ ID NO: 1 and SEQ ID NO: 2.

In the present invention, the composition may further comprise an agent capable of measuring a methylation level of the sequence represented by SEQ ID NO: 2. The agent capable of measuring the methylation level may be a primer, probe or antisense nucleic acid binding to the CpG site of the sequences represented by SEQ ID NO: 1 and SEQ ID NO: 2, and the primer, probe or antisense nucleic acid may be used as a hybridizable array element and may be fixed on a substrate.

The substrate is an appropriate solid or semi-solid supporter, and for example, it may comprise a film, a filter, a chip, a slide, a wafer, a fiber, a magnetic bead or non-magnetic bead, gel, tubing, a plate, a polymer, a microparticle and a capillary tube. The hybridizable array element may be fixed on the substrate by a chemical binding method, a covalent binding method such as UV or a linker (e.g.: ethylene glycol oligomer and diamine).

In one specific embodiment of the present invention, the DNA isolated from a biological sample (sample DNA) may be hybridized with the array element as being applied to hybridizable array, and the hybridization condition may be variously modified, and the detection and analysis of the hybridization level may be variously conducted according to the technology known in the art. In addition, in order to provide a signal allowing to confirm hybridization, the sample DNA and/or primer, probe or antisense nucleic acid may be labelled, and linked to oligonucleotide.

The label may comprise fluorophores (for example, fluorescein, phycoerythrin, rhodamine, lissamine, Cy3 and Cy5 (Pharmacia)), chromophores, chemical luminophores, magnetic particles, radioactive isotopes (P32 and S35), enzymes (alkaline phosphatase or horseradish peroxidase), cofactors, substrates for enzymes, heavy metals (for example, gold), antibodies, streptavidin, biotin, digoxigenin and haptene having a specific binding partner such as a chelating group, but not limited thereto.

In the present invention, the hybridization of the primer, probe or antisense nucleic acid and sample DNA depends on various factors such as reaction temperature, hybridization and washing time, buffer components and their pH and ion strength, length of the nucleotide, nucleotide sequence, amount of GC sequence, and the like. The detailed condition for the hybridization may refer to Joseph Sambrook, et al., MolecularCloning, A LaboratoryManual, Cold Spring Harbor Laboratory Press, Cold Spring Harbor, N.Y.(2001); and M.L.M. Anderson, NucleicAcid Hybridization, Springer-Verlag New York Inc. N.Y.(1999).

After the hybridization reaction, a hybridization signal generated though the hybridization reaction may be detected. For example, when the probe is labelled by enzyme, the hybridization may be confirmed by reacting the substrate of enzyme with the hybridization reaction product.

As the enzyme and substrate, peroxidase (for example, horseradish peroxidase) and chloronaphtol, aminoethylcarbozole, diaminobenzidine, D-luciferin, lucigenin (bis-N-methylacridinium nitrate), resorufin benzyl ether, luminol, Amplex red reagent (10-acetyl-3,7-dihydroxyphenoxazine), HYR (p-phenylenediamine-HCl and pyrocatechol), TMB (tetramethylbenzidine), ABTS (2,2'-Azine-di[3-ethylbenzthiazoline sulfonate]), o-phenylene diamine (OPD) and naphtol/pyronine; alkaline phosphatase and bromochloroindolyl phosphate (BCIP), nitroblue tetrazolium (NBT), naphthol-AS-B1-phosphate and ECF substrate; glucose oxidase and t-NBT (nitroblue tetrazolium) and m-PMS (phenzaine methosulfate) may be used.

Other aspect of the present invention provides a composition for diagnosing liver cancer comprising an agent capable of measuring a methylation level of the sequence represented by SEQ ID NO: 1.

In the present invention, the composition for diagnosing liver cancer may further comprise an agent capable of measuring a methylation level of the sequence represented by SEQ ID NO: 2, and the agent capable of measuring the methylation level comprises a primer, probe or antisense nucleic acid binding to CpG site of the sequences consisting of SEQ ID NO: 1 and SEQ ID NO: 2. The sequence represented by SEQ ID NO: 1 or 2 may be genome DNA, and may be a sequence in which non-methylated cytosine is converted into uracil by treating with bisulfite.

In the present invention, when the methylation level of a liver cancer tissue-specific marker is measured after separating DNA from a biological sample isolated from a subject, liver cancer tissue-derived DNA can be detected. As a result, liver cancer can be diagnosed by confirming presence of DNA derived from liver cancer tissue, and the composition for diagnosing liver cancer of the present invention is characterized in that it can diagnose liver cancer using biological samples of various origins.

In the present invention, the biological sample comprises tissue, tissue fragments, cells, cell fragments, blood, plasma, body fluids, feces and urine isolated from the subject, and the like, but not limited thereto.

### [ADVANTAGEOUS EFFECTS]

The method for determining whether a biological sample is originated from liver cancer tissue and composition for performing the same use a liver cancer tissue-specific DNA methylation marker, and the liver cancer tissue-specific marker has a low methylation level in normal liver tissue and other tissue, and has a high methylation level only in liver cancer tissue, and therefore, whether the biological sample is originated from liver cancer tissue can be determined with excellent accuracy.

### [BRIEF DESCRIPTION OF THE DRAWINGS]

FIG. 1 schematically shows the process of discovering a liver cancer tissue-specific marker.
FIG. 2 shows a variable importance plot (VarImp Plot) of the discovered liver cancer tissue-specific marker.

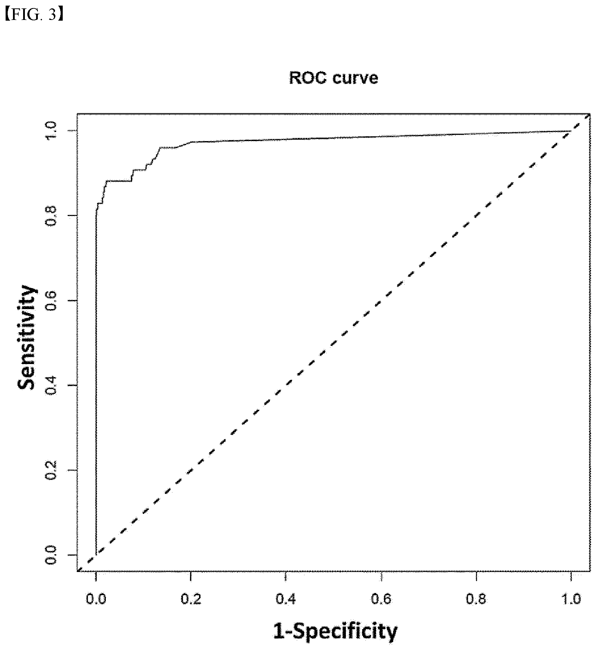

FIG. 3 shows the result of confirming the performance of the ultimately selected cg13204512 and cg00108164 markers.
FIG. 4 shows the result of confirming the methylation level of the ultimately selected cg13204512 and cg00108164 markers in the normal liver tissue (LIHC_N) and liver cancer tissue (LIHC_T), and pan-tumor tissue except liver (Pan_T).
FIG. 5 shows the result of confirming the methylation level of cg13204512 marker in various normal tissues (A) and cancer tissues (B).
FIG. 6 shows the result of confirming the methylation level of cg00108164 marker in various normal tissues (A) and cancer tissues (B).
FIG. 7 shows the result of confirming the methylation level of cg13204512 and cg00108164 markers in major normal tissues and cancer tissues: bladder (BL), breast (BR), cervix (CE), colon (CO), esophagus (ES), glioblastoma (GB), head and neck (HN), kidney (KI), liver (LI), lung (LU), pancreas (PA), paraganglioma (PC), prostate (PR), rectum (RE), sarcoma (SA), skin (SK), stomach (ST), thymus (TH), uterine (UC) and blood (B).

### [MODE FOR INVENTION]

Hereinafter, the present invention will be described in more detail by one or more specific examples. However, these examples are intended to illustrate one or more specific examples, but the scope of the present invention is not limited by these examples.

### Example 1: Discovering of liver cancer tissue-specific methylation markers

DNA methylation data for various carcinomas were downloaded from The Cancer Genome Atlas (hereinafter, referred to as TCGA). From the data downloaded from TCGA, methylated CpG sites were selected from liver cancer tissue samples (n=379), and non-methylated CpG sites were additionally selected from carcinoma samples of other tissues (n=7,260) among them. The criterion was to classify as methylated in case of 50% or more of samples were methylated, and unmethylated in case of 90% or more of samples were unmethylated.

Then, the non-methylated CpG sites in 90% of carcinoma samples (pan-tumor) other than liver cancer and CpG sites with different methylation levels of 30% or more in normal liver tissue and liver cancer tissue were additionally selected.

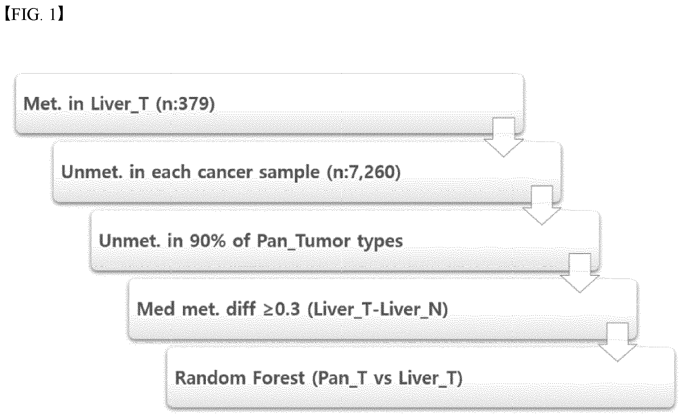

In FIG. 1, the process of discovering the liver cancer tissue-specific markers of the present invention was schematically shown.

### Example 2: Validation of performance of liver cancer tissue-specific methylation markers

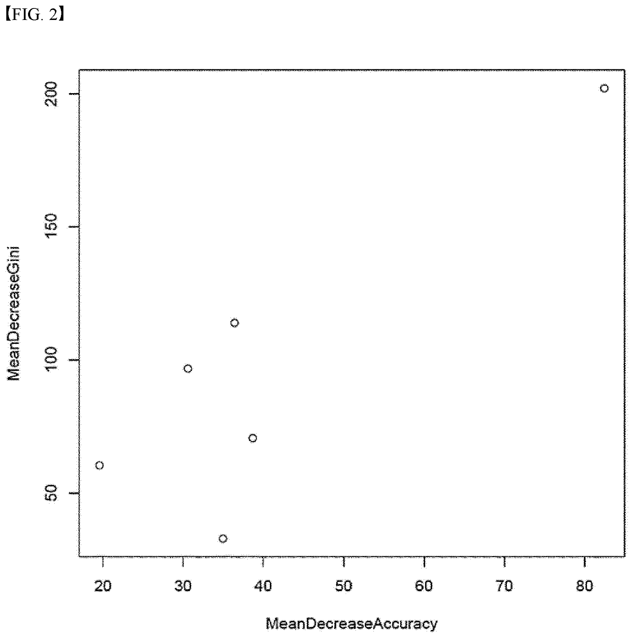

A variable importance plot (VarImp Plot) of the liver cancer tissue-specific markers discovered in Example 1 above was prepared by the random forest method, and this was shown in FIG. 2. In FIG. 2, MeanDecreaseAccuracy of the X-axis indicates the extent to which each marker contributes to improvement of accuracy in the classification of liver cancer tissue/normal liver tissue, and MeanDecreaseGini of the Y-axis indicates the extent to which each marker contributes to improvement of impurity in the classification of liver cancer tissue/normal liver tissue and other tissue. In other words, a larger value means that the marker can clearly distinguish liver cancer tissue, and normal liver tissue and other tissues.

Then, liver cancer tissue-specific markers were tested by randomly dividing the methylation data of cancer tissue used in Example 1 into training data and validation data. As a result, as shown in Table 1 below, it could be confirmed that the discovered markers distinguished liver tissue and other tissues with high efficiency and accuracy. The performance of the liver cancer tissue-specific markers was shown to have accuracy of 0.9915, sensitivity of 0.8816, specificity of 0.9973 and area under the curve (AUC) of 0.9668.

**[Table 1]**

| | **Type** | **Other cancer tissue** | **Liver cancer tissue** |
|---|---|---|---|
| **Training data** | Other cancer tissue | 5836 | 12 |
| | Liver cancer tissue | 57 | 246 |
| **Validation data** | Other cancer tissue | 1458 | 9 |
| | Liver cancer tissue | 4 | 67 |

Then, the performance of the two markers (cg13204512, cg00108164) with high importance in the variable importance plot of FIG. 1 was further validated. As a result, as shown in FIG. 2, the area under the curve (AUC) of two markers was 0.9725, confirming that the performance of distinguishing liver cancer tissue and other tissues was excellent.

Based on the above results, cg13204512 and cg00108164 markers were ultimately selected as liver cancer tissue-specific markers. In Table 2 and Table 3 below, the information and sequences of cg13204512 and cg00108164 markers were described.

**[Table 2]**

| **Probe_ID** | **CGRC_ID** | **Gene_ID** | **chr** | **start** | **end** | **CGI** | **CGI_loci** |
|---|---|---|---|---|---|---|---|
| cg13204512 | CGRC_HS.1 | RNF135 | chr17 | 29298184 | 29298185 | chr17:29298046-29298606 | pCGI |
| cg00108164 | CGRC_HS.2 | SH3YL1;ACP1 | chr2 | 264199 | 264200 | chr2:263400-265238 | pCGI |

**[Table 3]**

| **Probe_ID** | **Sequence** |
|---|---|
| cg13204512 | CGTGTGGCTGGCCGAGGACGACCTCGGCTGCATCATCTGCCAGGGGCT |
| | GCTGGACTGGCC[CG]CCACGCTGCCCTGCGGCCACAGCTTCTGCCGCC ACTGCCTGGAGGCCCTGTGGGGCGCCC (SEQ ID NO: 1) |
| **cg00108164** | GACAAAAACCACGCGCCCGCCGGGCCGCGCTCAGGCCTTCGCCCTCA GGGACTTCGGAAC[CG]CCCCGTCCTCAAGATCGAAAAGCCCAGAGCC CCGCGGCGGCTCCAAGCACGGTGTTGGGG (SEQ ID NO: 2) |

| | |
|---|---|
| - In the table, [CG] means a target methylation site of each probe. | |

### Example 3: Validation of finally selected liver cancer tissue-specific markers

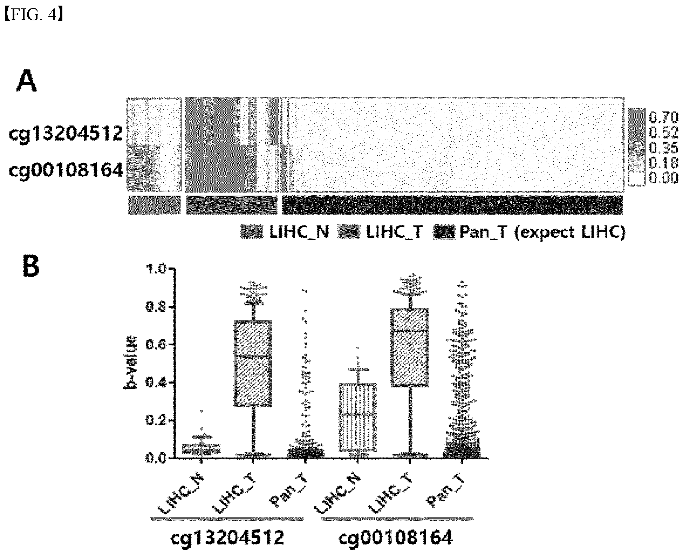

The methylation level of the finally selected cg13204512 and cg00108164 markers in the liver cancer tissue, normal liver tissue and other tissue was confirmed. As a result, as shown in A and B of FIG. 4, it could be seen that all the two markers were methylated at a low level in the normal liver tissue (LIHC_N) and pan-tumor tissue except liver (Pan_T), whereas they were methylated at a high level in the liver cancer tissue of the liver cancer tissue (LIHC_T).

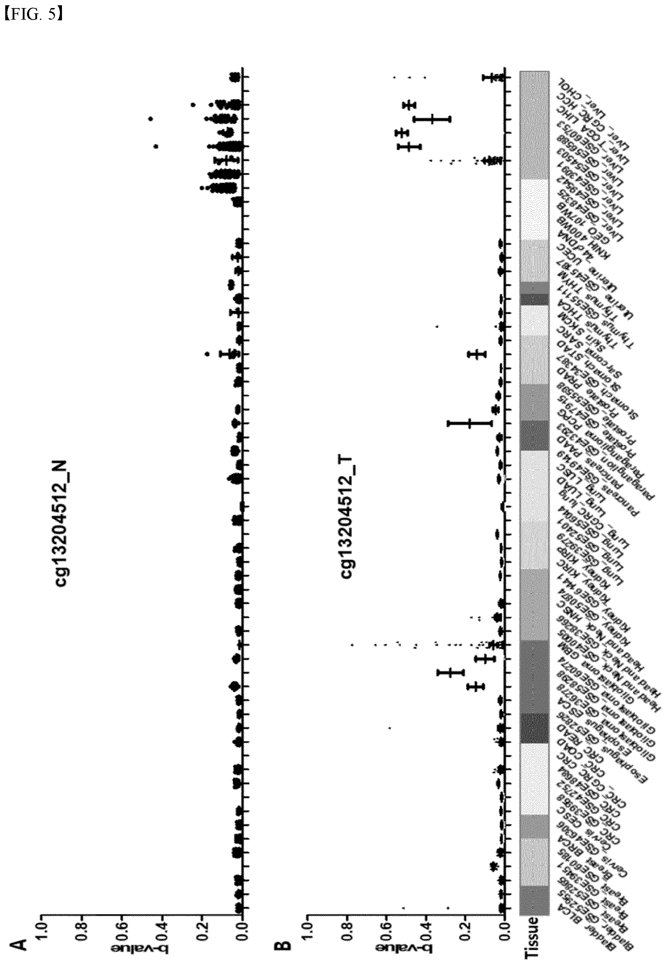

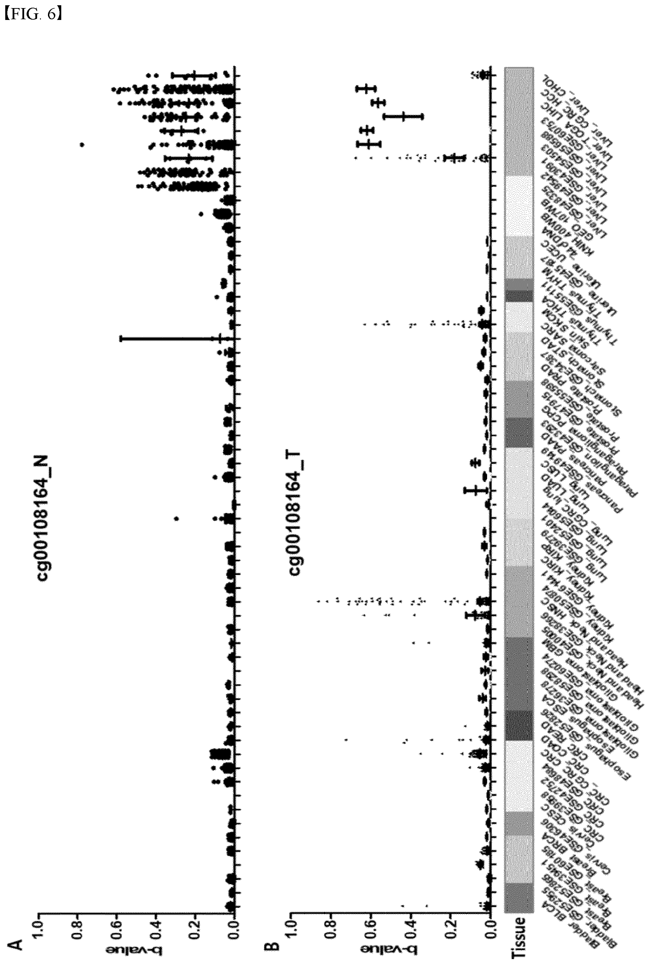

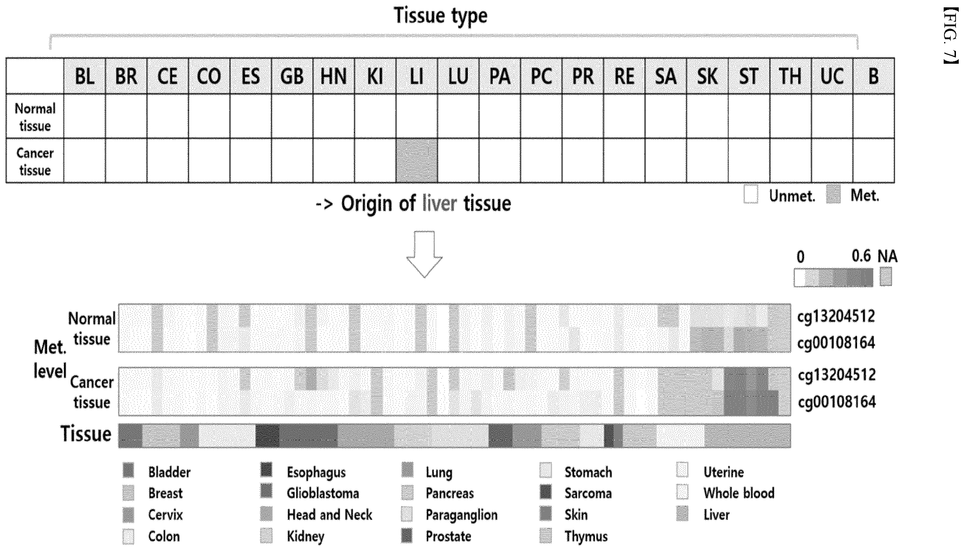

In FIG. 5 to FIG. 7, the methylation levels of cg13204512 and cg00108164 markers confirmed in the liver tissue and other tissues were shown.

From the results so far, it can be seen that cg13204512 and cg00108164 markers have a high methylation level in liver cancer tissue, and have a low methylation level in normal liver tissue and other tissues, and thus, the two markers can be used as a liver cancer tissue-specific marker.

## Claims

1. A method for determining whether a biological sample originates from liver cancer tissue comprising;
(a) separating DNA from a biological sample isolated from a subject; and
(b) measuring a methylation level of CpG site of the sequence represented by SEQ ID NO: 1 in the separated DNA.

2. The method for determining whether a biological sample originates from liver cancer tissue according to claim 1, wherein the (b) further measures a methylation level of CpG site of the sequence represented by SEQ ID NO: 2 in the separated DNA.

3. The method for determining whether a biological sample originates from liver cancer tissue according to claim 1, wherein the biological sample is selected from the group consisting of tissue, tissue fragments, cells, cell fragments, blood, plasma, body fluids, feces and urine isolated from the subject.

4. The method for determining whether a biological sample originates from liver cancer tissue according to claim 1,
wherein the (b) is performed by a method selected from the group consisting of PCR, methylation specific PCR, real time methylation specific PCR, MethyLight PCR, MehtyLight digital PCR, EpiTYPER, PCR using methylated DNA specific binding protein, quantitative PCR, DNA chip, molecular beacon, MS-HRM (Methylation-sensitive high resolution melting), asymmetric PCR, asymmetric PCR MS-HRMA (asymmetric PCR Methylation-sensitive high resolution melting analysis), Recombinase Polymerase Amplification, LAMP (Loop-Mediated Isothermal Amplification), Eclipse probe, next generation sequencing panel (NGS panel), pyrosequencing and bisulfide sequencing.

5. A composition for determining whether a biological sample originates from liver cancer tissue comprising an agent capable of measuring a methylation level of the sequence represented by SEQ ID NO: 1.

6. The composition for determining whether a biological sample originates from liver cancer tissue according to claim 5, wherein the composition further comprises an agent capable of measuring a methylation level of the sequence represented by SEQ ID NO: 2.

7. The kit for determining whether a biological sample originates from liver cancer tissue according to claim 5, wherein the agent capable of measuring the methylation level is a primer, probe or antisense nucleic acid binding to CpG site of the sequence consisting of SEQ ID NO: 1 and SEQ ID NO: 2.

8. A composition for diagnosing liver cancer comprising an agent capable of measuring a methylation level of the sequence represented by SEQ ID NO: 1.

9. The composition for diagnosing liver cancer according to claim 8, further comprising an agent capable of measuring a methylation level of the sequence represented by SEQ ID NO: 2.


<110>    LEPIDYNE CO., LTD.

<120>    Method for Identifying Whether Biological Sample is Derived from
         Liver Cancer Tissue

<130>    X20U16C0162

<150>    KR 1020190124552
<151>    2019-10-08

<160>    2

<170>    KoPatentIn 3.0

<210>    1
<211>    122
<212>    DNA
<213>    Artificial Sequence

<220>
<223>    polynucleotides


<400>    1
cgtgtggctg gccgaggacg acctcggctg catcatctgc caggggctgc tggactggcc        60

cgccacgctg ccctgcggcc acagcttctg ccgccactgc ctggaggccc tgtggggcgc        120

cc                                                                      122


<210>    2
<211>    122
<212>    DNA
<213>    Artificial Sequence

<220>
<223>    polynucleotides


<400>    2
gacaaaaacc acgcgcccgc cgggccgcgc tcaggccttc gccctcaggg acttcggaac        60

cgccccgtcc tcaagatcga aaagcccaga gccccgcggc ggctccaagc acggtgttgg       120

gg                                                                      122